# EUROPÄISCHE PATENTSCHRIFT

(11) **EP 1 615 628 B1**
(45) Veröffentlichungstag und Bekanntmachung des Hinweises auf die Patenterteilung: **03.12.2008**
(21) Anmeldenummer: 04726478.3
(22) Anmeldetag: 08.04.2004
(51) Int. Cl.: A61K 9/70

(54) **MEDIZINISCHE WIRKSTOFFPFLASTER MIT VERRINGERTER OPTISCHER AUFFÄLLIGKEIT AUF DER HAUT**
MEDICAL ACTIVE AGENT PATCH OPTICALLY LESS VISIBLE ON SKIN
DISPOSITIF TRANSDERMIQUE MEDICAL A PRINCIPE ACTIF VISUELLEMENT PLUS DISCRET SUR LA PEAU

(30) Priorität: 17.04.2003 DE 10317692
(43) Veröffentlichungstag der Anmeldung: 18.01.2006
(73) Patentinhaber: LTS LOHMANN Therapie-Systeme AG, 56626 Andernach (DE)
(72) Erfinder: BRACHT, Stefan, 07751 Cospeda (DE)
(74) Vertreter: Flaccus, Rolf-Dieter
(86) Internationale Anmeldenummer: PCT/EP2004/003748
(87) Internationale Veröffentlichungsnummer: WO 2004/091590

(56) Entgegenhaltungen:
- WO-A-01/78678
- WO-A-02/34200
- US-B1- 6 361 790

## Beschreibung

Die vorliegende Erfindung betrifft medizinische Wirkstoffpflaster, insbesondere transdermale therapeutische Systeme, mit ein- oder mehrschichtig aufgebauter, wirkstoffhaltiger Matrix, und einer mit dieser Matrix verbundenen Rückschicht, wobei sich diese Wirkstoffpflaster beim Tragen auf der Haut durch ein verbessertes optisches Erscheinungsbild auszeichnen.
Die Erfindung umfaßt ferner Verfahren, die die Herstellung derartiger Wirkstoffpflaster ermöglichen.

Viele der für die Herstellung von Wirkstoffpflastern oder TTS in Betracht kommenden Wirkstoffe oder Hilfsstoffe weisen bei der Lagerung eine Tendenz zur Verfärbung, beispielsweise zur Vergilbung, auf. Auch während der Applikationsdauer kann es zu derartigen nachteiligen Veränderungen kommen. Bekannt ist z. B. die bei Nikotinpflastern allmählich auftretende Gelbfärbung.

Die genannten Veränderungen beruhen zumeist auf oxidativen Zersetzungsprozessen, die insbesondere bei Lagerung oder während des Tragens des Wirkstoffpflasters auf der Haut im Kontakt mit Luftsauerstoff und Feuchtigkeit voranschreiten und durch Lichteinwirkung begünstigt werden können. Von solchen Prozessen betroffen sind insbesondere pharmazeutische Wirkstoffe, Antioxidantien, verschiedene Enhancer (d. h. Stoffe, die die transdermale Wirkstoffaufnahme unterstützen oder beschleunigen) sowie oxidationsempfindliche Bestandteile des im Wirkstoffpflaster vorhandenen Haftklebers, wie z. B. Klebharze.

Das Ausmaß der Zersetzung von Inhaltsstoffen muß sich nicht zwangsläufig nachteilig auf die pharmazeutische Qualität der Produkte auswirken, wenn beispielsweise die entstehenden Abbauprodukte nur Bruchteile eines Gewichtsprozentes der Ausgangsverbindung ausmachen, und wenn diese Abbauprodukte toxikologisch unbedenklich sind. So kommt es aufgrund der Verfärbungen häufig zu kosmetischen Produktbeeinträchtigungen, ohne daß bereits eine Beeinträchtigung der pharmazeutischen Qualität stattgefunden hat. Diese nachteiligen Veränderungen des optischen Erscheinungsbildes der Wirkstoffpflaster werden von den Anwendern oder Patienten insbesondere im Falle von Arzneimitteln häufig mit Schadhaftigkeit oder Verderb in Verbindung gebracht, wodurch eine Verunsicherung entsteht.
Häufig handelt es sich bei diesen Veränderungen um Gelb-, Braun- oder Rotverfärbungen, wie sie typischerweise bei chemischen Zersetzungen auftreten. Bereits geringfügige Farbveränderungen können von den Anwendern oder Patienten als Anzeichen für eine Verschlechterung der Qualität des Arzneimittels interpretiert werden.

Die Problematik der Verfärbungen tritt insbesondere dann auf, wenn das Produkt im Zustand frisch nach der Herstellung dem menschlichen Auge zunächst farblos oder weiß erscheint und die erwähnten Verfärbungen erst nach einer gewissen Zeit der Lagerung oder während des Tragens eines Pflasters auf der Haut in Erscheinung treten. Dies wird von den Anwendern in noch höherem Maße als kritisch oder potentiell gefährlich wahrgenommen als eine von Anfang an vorhandene Färbung des Produkts, die sich während der Lagerung lediglich verstärkt.

Im Bereich medizinischer Wirkstoffpflaster stellen transparente und farblose Pflaster in kosmetischer Hinsicht den Idealfall dar, da sie auf der Haut des Anwenders von diesem selbst oder von anderen Personen als unauffällig empfunden werden. Die Anwender medizinischer Pflaster bevorzugen in der Regel Pflaster mit solchen unauffälligen Eigenschaften, weil auf diese Weise das Risiko vermindert wird, daß andere Personen auf die Behandlungsbedürftigkeit des Anwenders aufmerksam gemacht werden und möglicherweise von der zugrunde liegenden Krankheit des Anwenders erfahren.

Transparante und transluzente Wirkstoffpflaster sind aus WO 01/78678, WO 02/34200 und US 6,361,790 bekannt.

Falls eine transparente Ausführung eines Wirkstoffpflasters beispielsweise wegen vorhandener Färbungen der Inhaltsstoffe oder wegen während der Lagerzeit auftretender Verfärbungen aus kosmetischen Gründen nicht sinnvoll ist, so kann das Pflaster mit einer undurchsichtigen Rückschicht ausgestattet werden. Während der Applikationsdauer wird dann durch diese Rückschicht die optische Wahrnehmung der Färbung oder Verfärbung verhindert.

Nachteilig wirkt sich im letztgenannten Fall aber aus, daß die mit einer undurchsichtigen Rückschicht ausgestatteten Pflaster oder TTS am Applikationsort, d. h. auf der Haut des Patienten, wesentlich auffälliger in Erscheinung treten als transparente oder farblose Pflaster. Eine im Stand der Technik bekannte und häufig angewandte Maßnahme besteht darin, die undurchsichtige Rückschicht hautfarben zu lackieren. Hier ergibt sich jedoch ein weiteres Problem, da es sich nämlich als äußerst schwierig erweist, einen Hautton zu finden, der für eine größere Anzahl von Anwendern, die unterschiedliche Hautfärbungen haben, gleichermaßen passend und in kosmetischer Hinsicht annehmbar ist. Gänzlich unmöglich ist es, unter Einbeziehung aller Hauttypen der Weltbevölkerung einen einheitlichen, opaken Hautfarbton festzulegen, der sich als Farbton für eine undurchsichtige Rückschicht eignen könnte. Zwar könnte dieses Problem dadurch gelöst werden, daß ansonsten identische Wirkstoffpflaster mit unterschiedlich gefärbten Rückschichten, abgestimmt auf die unterschiedlichen Hautfarbtöne der Weltbevölkerung, hergestellt werden. Dies kommt jedoch wegen der komplexen Herstell- und Vertriebslogistik und letztlich aus Kostengründen nicht in Frage.

Aufgabe der vorliegenden Erfindung war es deshalb, Wirkstoffpflaster bereitzustellen, die trotz vorhandener oder im Lauf der Zeit auftretender Färbungen ein optisch unauffälliges Erscheinungsbild des Pflasters gewährleisten, insbesondere, wenn sich das Pflaster am Applikationsort befindet. Dabei soll möglichst eine einheitliche Lösung gefunden werden, die für die unterschiedlichsten Hautfarbtöne der Weltbevölkerung geeignet ist.
Ferner lag der Erfindung die Aufgabe zugrunde, Verfahren aufzuzeigen, durch welche derartige Wirkstoffpflaster erhalten werden können.

Diese Aufgaben werden durch medizinische Wirkstoffpflaster gemäß Anspruch 1 sowie durch Herstellungsverfahren gemäß Anspruch 11 gelöst, sowie durch die in den abhängigen Ansprüchen beschriebenen Ausführungsformen.

Demnach treten die oben genannten Nachteile bei den im Oberbegriff des Anspruchs 1 beschriebenen medizinischen Wirkstoffpflastern nicht oder nur in abgeschwächter Form auf, wenn dieses Wirkstoffpflaster transparent oder zumindest transluzent ist und wenn das Pflaster - im Zustand der Applikation auf der Haut einer ersten Person - an einer mit dem Pflaster bedeckten Hautstelle einen Helligkeits-Farbwert L₁ aufweist, der nicht weniger als 50% und nicht mehr als 200% eines Helligkeits-Farbwertes L₂ beträgt, wobei L₂ der Helligkeitswert an der das applizierte Pflaster umgebenden Hautregion derselben Person ist, und wenn dasselbe in Bezug auf die Haut einer zweiten oder einer jeden weiteren Person gilt, sofern L₂ bei allen genannten Personen im Bereich von 5° und 100°, insbesondere im Bereich von 20° und 90°, liegt. Die genannten Unterschiede zwischen den Helligkeitswerten L₁, L₂ können durch Messungen bei repräsentativen Stichproben von Personen des jeweiligen Hauttyps ermittelt werden.

Der Farbwert der Helligkeit L, genannt "Helligkeits-Farbwert", ist eine farbmetrische Kenngröße, die in Verbindung mit weiteren Kenngrößen in der Technik zur eindeutigen Charakterisierung von Farben verwendet wird. Der Helligkeits-Farbwert wird in Grad angegeben und kann durch Farbmeßgeräte bestimmt werden. Die hier angegebenen Werte für die Farbhelligkeit wurden mit Hilfe eines "Dreibereichsfarbmeßgerätes CP-320" der Fa. Techkon GmbH (DE-61462 Königstein) ermittelt.

Überraschenderweise hat sich herausgestellt, daß Wirkstoffpflaster mit den vorgenannten erfindungsgemäßen Merkmalen ein unauffälliges Erscheinungsbild am Applikationsort, d. h. auf der Haut, aufweisen, und daß derartige Wirkstoffpflaster auf unterschiedlichsten Hautfarbtypen der Weltbevölkerung optisch unauffällig erscheinen. Beispielsweise hat ein erfindungsgemäßes Wirkstoffpflaster ein gleichermaßen unauffälliges optisches Erscheinungsbild, wenn es auf die Haut eines Anwenders mit kaukasischer, heller Hautfarbe oder auf die Haut eines Anwenders mit dunkler, negroider Hautfarbe appliziert wird. Deshalb zeichnet sich nach einer bevorzugten Ausführungsform ein erfindungsgemäßes Wirkstoffpflaster dadurch aus, daß der an der nicht mit dem Pflaster bedeckten Hautstelle gemessene Helligkeits-Farbwert L₂ der genannten ersten Person der Helligkeits-Farbwert einer Person mit heller, kaukasischer Hautfarbe ist, und daß der Helligkeitsfarbwert L₂ der genannten zweiten Person der Helligkeits-Farbwert einer Person mit dunkler, negroider Hautfarbe ist, oder umgekehrt.

Die Herstellung von Wirkstoffpflastern mit den Merkmalen des Oberbegriffs des Anspruchs 1 und die zur Herstellung geeigneten Stoffe sind dem Fachmann grundsätzlich bekannt. Zur Herstellung der Matrixschicht(en) können beispielsweise Stoffe aus der Gruppe der Polyacrylate, Poly(meth)acrylate, Klebharze, Cellulosederivate, Polyisobutylene, Styrol-Isopren-Styrol-Blockcopolymere, Styrol-Butadien-Styrol-Blockcopolymere, Polydimethylsiloxane, Ethylenvinylacetat-Copolymere und Vinylacetat verwendet werden, wahlweise unter Hinzufügung von dem Fachmann bekannten Hilfsstoffen. Mindestens eine der Matrixschichten enthält einen Wirkstoff, wobei insbesondere ein Arzneistoff zu verstehen ist, oder mehrere solcher Wirkstoffe.

Die erfindungsgemäßen, aus einer Matrix und darüber liegender Rückschicht aufgebauten Wirkstoffpflaster sind im wesentlichen transparent oder zumindest transluzent (d. h. durchscheinend), jedenfalls nicht opak. Demzufolge ist auch die Rückschicht im wesentlichen transparent oder transluzent.

Als Rückschicht eignen sich vor allem Polyester, wie z. B. Polyethylenterephthalat (PET) und Polybutylenterephthalat, darüber hinaus aber auch nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr.

Die erfindungsgemäßen Wirkstoffpflaster enthalten in mindestens einer ihrer Schichten einen oder mehrere Stoffe aus der Gruppe der Farbstoffe und Pigmente. Dadurch wird in Kombination mit den transparenten oder transluzenten Eigenschaften des Pflasters erreicht, daß eine von Anfang an bestehende Färbung des/der Inhaltsstoffe der Matrix oder eine erst nach Ende der Herstellung beginnende und fortschreitende Verfärbung dieser Stoffe optisch maskiert wird. Zugleich wird dadurch die Färbung ausreichend an den Hautton der Applikationsstelle adaptiert oder angepaßt, so daß das Pflaster auf den unterschiedlichsten Hautfarbtypen unauffällig erscheint. Vorzugsweise ist/sind der/die zur optischen Maskierung eingesetzten Stoff(e), ausgewählt aus der Gruppe der Farbstoffe und Pigmente, in der Matrixschicht oder in mindestens einer der Matrixschichten eines mehrschichtigen Pflasters enthalten.

Nach einer weiteren, besonders bevorzugten Ausführungsform wird die optische Maskierung dadurch bewirkt, daß die transparente oder transluzente Rückschicht einen Gehalt an mindestens einem Stoff, ausgewählt aus der Gruppe der Farbstoffe und Pigmente, aufweist. Dies kann nach insbesondere dadurch bewerkstelligt werden, daß die Rückschicht des Pflasters auf der Außenseite, d. h. auf der der Haut abgewandten Seite, mit einer Beschichtung oder einem Lack überzogen wird, die/der mindestens einen Farbstoff oder/und mindestens ein Farbpigment enthält. Diese Variante hat den zusätzlichen Vorteil, daß die Einfärbung weder mit der Haut noch mit der wirkstoffhaltigen Matrix in Berührung kommen kann.

Ferner kann es vorteilhaft sein, wenn sowohl die Matrixschicht(en) als auch die Rückschicht einen Gehalt an Farbstoff(en) oder/und Pigment(en) aufweisen.

Überraschenderweise hat sich gezeigt, daß zur Erzielung einer adaptiven Wirkung weniger die Angleichung der Farbstoffe oder Pigmente an den jeweiligen Hautton ausschlaggebend ist, sondern daß diese Wirkung im wesentlichen durch die eingesetzte(n) Konzentration(en) der Farbstoffe oder/und Pigmente bestimmt wird. Die optische Auffälligkeit eines Wirkstoffpflasters wird im wesentlichen durch die Konzentrationen der darin enthaltenen Farbstoffe und Pigmente bestimmt, wobei die Schichtdicke des Pflasters zusätzlich zu berücksichtigen ist. Damit das Pflaster im Auge eines Beobachters als unauffällig erscheint, dürfen bestimmte Konzentrationen der enthaltenen Farbstoffe oder/und Pigmente (einschließlich der gefärbten oder verfärbten Inhaltsstoffe, insbesondere Wirkstoffe) nicht überschritten werden. Diese Konzentrationen lassen sich anhand der in Anspruch 1 genannten Bedingungen ermitteln.

Bei niedrigen Konzentrationen an gefärbten oder verfärbten Inhaltsstoffen in der Matrix können auch solche Farben oder Farbpigmente noch optisch unauffällig wirken, die von dem Farbton der darunter liegenden Haut an der Applikationsstelle deutlich verschieden sind. Dasselbe gilt, wenn das Pflaster eine geringe Schichtdicke aufweist. Durch die niedrige Konzentration und/oder die geringe Schichtdicke, ergibt sich ein Konzentrations-Spielraum oder entsprechende Variationsmöglichkeiten für die Schichtdicke, wodurch die Voraussetzungen für eine optische Maskierung von Verfärbungen von Inhaltsstoffen von Wirkstoffpflastern durch Beimischung von Farbstoffen oder/und Pigmenten gegeben sind.

Eine weitere Verbesserung des optischen Erscheinungsbildes von auf die Haut applizierten Wirkstoffpflastern kann dadurch erreicht werden, daß gemäß einer besonders bevorzugten Ausführungsform zumindest die der Haut abgewandte Oberfläche der Rückschicht mit verminderten Reflexionseigenschaften versehen wird. Dies kann entweder mittels physikalischer Methoden geschehen, oder durch Aufbringen einer Antireflex-Schicht oder -Beschichtung. Eine solche Schicht oder Beschichtung enthält vorzugsweise ein optisches Mattierungsmittel oder eine Kombination von mindestens zwei Mattierungsmitteln. Diese Antireflex-Schicht kann zugleich Farbstoff(e) oder/und Pigment(e) zur Maskierung der Inhaltsstoffe des Pflasters enthalten, wie oben beschrieben.

Durch die Mattierung kann zusätzlich diejenige Ursache der optischen Auffälligkeit eines Wirkstoffpflasters beseitigt oder abgeschwächt werden, die auf Lichtreflexen beruht. Solche Lichtreflexe treten häufig bei Wirkstoffpflastern auf, die mit einer transparenten Rückschicht mit glatter Oberflächenstruktur versehen sind. Die Reflexionseigenschaften dieser Rückschicht-Materialien unterscheiden sich sehr stark von den Reflexionseigenschaften der menschlichen Haut, weshalb derartige Pflaster auf der Haut visuell deutlich auffallen.

Die Wirkstoffpflaster der vorliegenden Erfindung sind insbesondere dann vorteilhaft, wenn mindestens eine Schicht der Matrix einen oder mehrere gefärbte Inhaltsstoff(e) aufweist. Dabei kann es sich insbesondere um einen Stoff oder um Stoffe handeln, der/die im Ausgangszustand farblos ist/sind und während der Lagerung oder während der Applikationsdauer zur Verfärbung neigt/neigen oder sich verfärbt/verfärben. Besonders bevorzugt sind Wirkstoffpflaster, die als gefärbte(n) oder zu Verfärbung neigende(n) Inhaltsstoff(e) einen oder mehrere pharmazeutische Wirkstoffe enthalten, wobei Nicotin besonders bevorzugt ist.

Vorzugsweise handelt es sich bei den genannten Wirkstoffpflastern um transdermale therapeutische Systeme. Diese zeichnen sich dadurch aus, daß sie über eine bestimmte Zeit eine gleichbleibende Abgabe von Arzneistoffen über die Haut ermöglichen. Der Aufbau und die Herstellung derartiger Systeme sind dem Fachmann grundsätzlich bekannt.

Die Erfindung erstreckt sich ferner auf Verfahren zur Herstellung der vorstehend beschriebenen Wirkstoffpflaster. Diese Verfahren weisen folgende Schritte auf:
a) Herstellung eines Systems, welches eine ein- oder mehrschichtige wirkstoffhaltige Matrix und eine damit verbundene Rückschicht umfaßt, wobei die Matrix unter Verwendung von Matrixpolymer(en), Wirkstoff(en) und Hilfsstoffen hergestellt wird, und zu der Matrix oder/und der Rückschicht ein oder mehrere aus der Gruppe der Farbstoffe und Pigmente ausgewählte(r) Stoff(e) beigemischt wird/werden;
b) Herstellung mindestens eines weiteren Systems nach Schritt (a), wobei sich dieses System hinsichtlich der Konzentration der Farbstoffe oder/und Pigmente, und/oder hinsichtlich der Art der verwendeten Farbstoffe oder/und Pigmente unterscheidet;
c) Herstellung von Flächenabschnitten oder Stanzlingen aus den in Schritten (a) und (b) erhaltenen Systemen;
d) Herstellung oder Bereitstellung von Farbtafeln, die Helligkeits-Farbwerte L₂ im Bereich von 5° und 100°, insbesondere im Bereich von 20° und 90°, aufweisen;
e) Aufbringen oder Aufkleben der in Schritt (b) erhaltenen Abschnitte oder Systeme auf die unter (c) genannten Farbtafeln;
f) Messung der Farbwerte der Helligkeit L₁ der auf den Farbtafeln befindlichen Systeme und Bestimmung der Differenz zwischen L₂ und L₁ in jedem einzelnen Fall;
g) Auswahl derjenigen Systeme, deren Farbwert der Helligkeit L₁ nicht weniger als 50% und nicht mehr als 200% des Helligkeits-Farbwertes L₂ beträgt.

Durch die Lehre der vorliegenden Erfindung wird die Herstellung von Wirkstoffpflastern ermöglicht, welche trotz eines Gehalts an gefärbten oder sich verfärbenden Inhaltsstoffen beim Tragen auf der Haut für einen Beobachter nicht leicht zu erkennen sind und optisch nicht auffallen, und zwar unabhängig davon, ob das Pflaster auf der Haut eines hellhäutigen oder eines dunkelhäutigen Menschen befestigt ist.

Die Erfindung wird anhand der nachfolgenden Beispiele näher erläutert.

### Beispiele

### 1. Herstellung von Rückschichten mit unterschiedlichen Pigment-Konzentrationen

Aus Ethylcellulose und unterschiedlichen Anteilen eines Pigmentgemisches (s. Tabelle 1) wurden Beschichtungsmassen hergestellt und diese mittels einer Rakel auf eine PET-Folie von 15 µm Dicke beschichtet (Flächengewicht 7 - 10 g/m²).

**Tabelle 1:**

| Nr. | Ethylcellulose [Gew.-%] | Pigmentgemisch [Gew.-%] |
|---|---|---|
| 1 | 99,75 | 0,25 |
| 2 | 99,5 | 0,5 |
| 3 | 99,0 | 1,0 |
| 4 | 98,0 | 2,0 |
| 5 | 96,0 | 4,0 |

### Pigmentgemisch:

50,0 Gew.-% Naturell BB Plv Pigment
50,0 Gew.-% Naturell Pulver Pigment
(Fa. Cosnaderm Chemische Rohstoffe GmbH, D-68526 Ladenburg)

Als Kontroll-Beispiele wurden verwendet:
(6) PET-Folie, aluminisiert und nicotinbeständig (undurchsichtig)
(7) PET-Folie, 15 µm, transparent.
(8) Scotchpak 1006

### 2. Herstellung von Hautpflastern

Unter Verwendung der unter 1. genannten Rückschichten wurden Hautpflaster hergestellt. Hierzu wurde Durotak^{®} 2052 ((Fa. National Starch & Chemical B.V.) mit einem Flächengewicht von 80 g/m² ausgestrichen und jeweils mit einer der unter 1. genannten Rückschichten bedeckt. Nachfolgend wurden einzelne Pflaster mit einer Größe von jeweils 1 cm² ausgestanzt.

### 3. Herstellung von Farbtafeln, die den menschlichen Hautfarben entsprechen

Mit Hilfe der Software "PowerPoint" (Microsoft) und eines Farbdruckers (HP-C LaserJet 4500; Hewlett-Packard) wurden acht Farbtafeln erstellt, welche die verschiedenen Hautfarbtöne der Weltbevölkerung repräsentieren.

Die Farbtöne der Farbtafeln sind in "PowerPoint" durch die sechs Parameter Farbton, Rot, Grün, Blau, Sättigung, Intensität wie folgt charakterisiert und lassen sich anhand dieser Parameter reproduzieren:

**Tabelle 2:**

| Farbtafel Nr. | Farbton | Rot | Grün | Blau | Sättigung | Intensität |
|---|---|---|---|---|---|---|
| A | 16 | 255 | 215 | 191 | 255 | 223 |
| B | 21 | 50 | 25 | 0 | 255 | 25 |
| C | 21 | 80 | 40 | 0 | 255 | 40 |
| D | 21 | 255 | 236 | 217 | 255 | 236 |
| E | 21 | 197 | 137 | 77 | 130 | 137 |
| F | 21 | 72 | 36 | 0 | 255 | 36 |
| G | 21 | 117 | 78 | 39 | 128 | 78 |
| H | 25 | 255 | 226 | 183 | 255 | 219 |

Die Farbtafeln Nr. A - H wurden mit einem "Drei-Bereichsfarbmeßgerät CP-320" der Fa. Techkon GmbH (DE-61462 Königstein) vermessen. Dabei werden die Werte (in Grad) für die Helligkeit L, die Rot-Grün-Achse a und die Celb-Blau-Achse b ermittelt. Es wurden pro Farbtafel 10 Messungen durchgeführt und die Mittelwerte gebildet. Die Mittelwerte sind in der nachfolgenden Tabelle 2 dargestellt.

**Tabelle 3:**

| Farbtafel Nr. | L-Wert (L₂) | a-Wert | b-Wert |
|---|---|---|---|
| A | 82,464 | 10,986 | 13,634 |
| B | 21,791 | -3,203 | 8,877 |
| C | 25,776 | 5,905 | 14,758 |
| D | 88,086 | 4,945 | 9,572 |
| E | 50,596 | 10,893 | 36,304 |
| F | 25,811 | 3,747 | 12,968 |
| G | 32,562 | 5,519 | 21,015 |
| H | 83,228 | 6,712 | 24,95 |
| Mittelwert* | 51,289 | 5,688 | 17,758 |

| | | | |
|---|---|---|---|
| * Hierbei handelt es sich jeweils um den Mittelwert, der aus den Werten der 8 Farbtafeln gebildet wurde. | | | |

Wie ersichtlich, variiert der Farbwert der Helligkeit L am stärksten, wohingegen der a-Wert nur geringfügig unterschiedlich ist.

Die Palette der Hautfarben, bei denen das Prinzip der vorliegenden Erfindung vorteilhaft eingesetzt werden kann, umfaßt nach dem oben beschriebenen "L,a,b"-System insbesondere den Bereich von "5,8,60" bis "100,4,0".

### 4. Bestimmung der helligkeitswert-Unterschiede

Die unter 2. beschriebenen Hautpflaster-Stanzlinge wurden auf die unter 3. beschriebenen Farbtafeln aufgeklebt. Anschließend wurden mit dem unter 3. beschriebenen Meßverfahren die Helligkeits-Farbwerte L1 der aufgeklebten Pflaster bestimmt. Aus den erhaltenen Meßwerten L₁ wurde jeweils der Differenzbetrag zum Helligkeitswert L₂ des jeweiligen Untergrundes (d. h. der Farbtafel) gebildet. Die prozentualen Unterschiede zwischen den Helligkeits-Farbwerten L₁ der auf die Farbtafeln A bis H aufgeklebten Pflastertypen (Nr. 1 bis 5 und Kontrollen Nr. 6 bis 8) einerseits und den Helligkeitswerten L₂ der jeweiligen Farbtafeln A bis H sind in Tabelle 4 dargestellt.

Hieraus ist ersichtlich, daß die transparente PET-Folie (7) auf allen Farbtafeln die geringsten Abweichungen bezüglich des Helligkeitswertes aufweist (Positiv-Kontrolle). Umgekehrt werden bei den Kontrollbeispielen (6) und (8) die stärksten Abweichungen festgestellt.

### 5. Visuelle Auswertung

Da bekannt ist, daß die Farbempfindung des Menschen von den colorimetrisch bestimmten Daten abweichen kann, wurde eine visuelle Bewertung der auf die Farbtafeln A bis H aufgeklebten Test-Pflaster, einschließlich der Vergleichsbeispiele 6 bis 8, durch Versuchspersonen durchgeführt.

Hierzu wurde eine bestimmte Anzahl (z. B. 10) eines jeden Testpflasters (1 bis 8) auf Farbtafeln A bis H aufgeklebt. Diese Farbtafeln wurden unter standardisierten Bedingungen (Beleuchtung, Abstand, Beobachtungszeit) einer Gruppe von Testpersonen präsentiert. Die Anzahl der von den Versuchspersonen nicht wahrgenommenen Pflaster dient - nach statistischer Auswertung der Daten - als Maß für die optische Unauffälligkeit bzw. die Wirksamkeit der optischen Maskierung eines Pflasters.

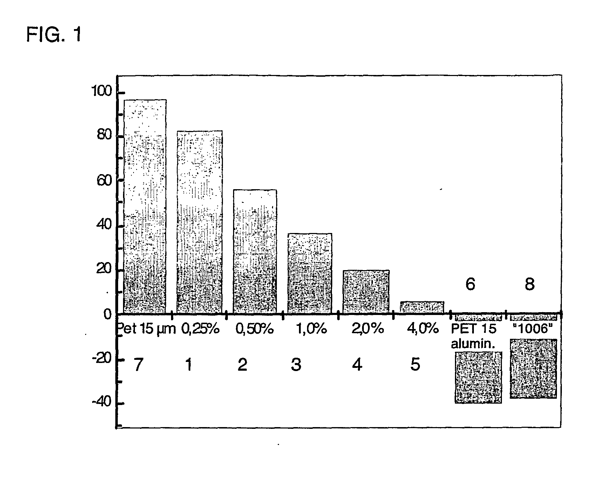

In Abbildung 1 sind die einzelnen Testpflaster Nr. 1 - 8 in Form eines Balkendiagramms in der Reihenfolge ihrer visuellen Unauffälligkeit (vertikale Achse) dargestellt. Pflaster Nr. 1 und Kontrollpflaster Nr. 7 waren auf den meisten Farbtafeln nicht oder kaum erkennbar.

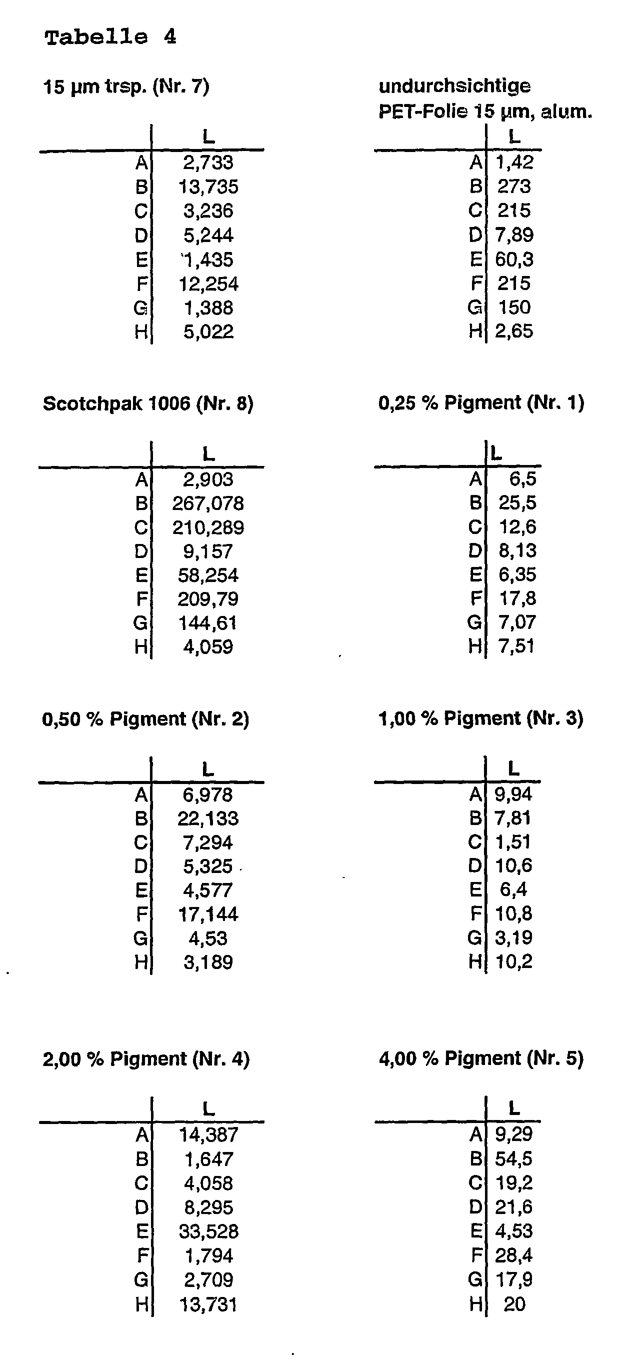

## Patentansprüche

1. Medizinisches Wirkstoffpflaster, das eine ein- oder mehrschichtig aufgebaute Matrix sowie eine mit dieser Matrix verbundene Rückschicht aufweist, wobei mindestens eine Schicht der Matrix wirkstoffhaltig ist, und wobei das Wirkstoffpflaster **dadurch gekennzeichnet ist,**
- **daß** mindestens eine Schicht der Matrix einen oder mehrere Inhaltsstoff(e) enthält, der/die eine Färbung aufweist/ aufweisen, oder der/die im Ausganaszustand farblos ist/ sind und während der Lagerung oder während der Aipplikationsdauer zur Verfärbung neiat/neiaen oder sich verfärbt/ verfärben,
- **daß** es transparent oder zumindest transluzent ist,
- in mindestens einer der genannten Schichten einen oder mehrere aus der Gruppe der Farbstoffe und Pigmente ausgewählte(n) Stoff(e) enthält,
- **daß** das Pflaster im Zustand der Applikation auf der Haut einer ersten Person an einer mit dem Pflaster bedeckten Hautstelle einen Helligkeits-Farbwert L1 aufweist, der nicht weniger als 50% und nicht mehr als 200% eines Helligkeits-Farbwertes L2 beträgt, wobei L2 der Helligkeitswert an der das applizierte Pflaster umgebenden Hautregion derselben Person ist, und
- **daß** dasselbe in Bezug auf die Haut einer zweiten oder einer jeden weiteren Person gilt, sofern L2 bei allen genannten Personen im Bereich von 5° und 100°, insbesondere im Bereich von 20° und 90°, liegt.

2. Wirkstoffpflaster nach Anspruch 1, **dadurch gekennzeichnet, daß** der Helligkeits-Farbwert L2 der genannten ersten Person der Helligkeits-Farbwert einer Person mit heller, kaukasischer Hautfarbe ist, und daß der Helligkeitsfarbwert L2 der genannten zweiten Person der Helligkeits-Farbwert einer Person mit dunkler, negroider Hautfarbe ist, oder umgekehrt.

3. Wirkstoffpflaster nach Anspruch 1 oder 2, **dadurch gekennzeichnet, daß** es den/die genannten, aus der Gruppe der Farbstoffe und Pigmente ausgewählte(n) Stoff(e), in der Matrixschicht oder in mindestens einer der Matrixschichten enthält.

4. Wirkstoffpflaster nach einem der vorangehenden Ansprüche, **dadurch gekennzeichnet, daß** die Rückschicht des Pflasters auf der der Haut abgewandten Seite mit einer Farbstoff(e) oder/und Farbpigment(e) enthaltenden Beschichtung, insbesondere einem Lack, überzogen ist.

5. Wirkstoffpflaster nach einem der vorangehenden Ansprüche, **dadurch gekennzeichnet, daß** zumindest die der Haut abgewandte Oberfläche der Rückschicht verminderte Reflexionseigenschaften hat.

6. Wirkstoffpflaster nach Anspruch 5, **dadurch gekennzeichnet, daß** die Verminderung der Reflexionseigenschaften durch physikalische Methoden bewirkt ist.

7. Wirkstoffpflaster nach einem der vorangehenden Ansprüche, **dadurch gekennzeichnet, daß** auf der der Haut abgewandten Seite der Rückschicht eine Antireflexschicht aufgebracht ist, welche vorzugsweise ein optisches Mattierungsmittel oder eine Kombination von mindestens zwei optischen Mattierungsmitteln enthält.

8. Wirkstoffpflaster nach Anspruch 7, **dadurch gekennzeichnet, daß** die Antireflexschicht zusätzlich mindestens einen Stoff enthält, der aus der Gruppe der Farbstoffe und Pigmente ausgewählt ist.

9. Wirkstoffpflaster nach einem der vorangehenden Ansprüche, **dadurch gekennzeichnet, daß** der genannte Inhaltsstoff, der im Ausgangszustand farblos ist und während der Lagerung oder während der Applikationsdauer zur Verfärbung neigt oder sich verfärbt, ein pharmazeutischer Wirkstoff, insbesondere Nicotin, ist.

10. Wirkstoffpflaster nach einem der vorangehenden Ansprüche, **dadurch gekennzeichnet, daß** es ein transdermales therapeutisches System ist.

11. Verfahren zur Herstellung eines Wirkstoffpflasters nach einem der vorangehenden Ansprüche, wobei das Verfahren folgende Schritte aufweist:
a) Herstellung eines Systems, umfassend eine ein- oder mehrschichtige wirkstoffhaltige Matrix und eine damit verbundene Rückschicht, wobei die Matrix unter Verwendung von Matrixpolymer(en), Wirkstoff(en) und Hilfsstoffen hergestellt wird, und in die Matrix oder/und die Rückschicht ein oder mehrere aus der Gruppe der Farbstoffe und Pigmente ausgewählte(r) Stoff(e) eingearbeitet wird;
b) Herstellung mindestens eines weiteren Systems nach Schritt (a), wobei sich dieses System hinsichtlich der Konzentration der Farbstoffe oder/und Pigmente, und/oder hinsichtlich der Art der verwendeten Farbstoffe oder/und Pigmente unterscheidet;
c) Herstellung von Flächenabschnitten oder Stanzlingen aus den in Schritten (a) und (b) erhaltenen Systemen;
d) Herstellung oder Bereitstellung von Farbtafeln, die Helligkeits-Farbwerte L2 im Bereich von 5° und 100°, insbesondere im Bereich von 20° und 90°, aufweisen;
e) Aufbringen oder Aufkleben der in Schritt (b) erhaltenen Abschnitte oder Systeme auf die unter (c) genannten Farbtafeln;
f) Messung der Farbwerte der Helligkeit L₁ der auf den Farbtafeln befindlichen Systeme und Bestimmung der Differenz zwischen L₂ und L₁ in jedem einzelnen Fall;
g) Auswahl derjenigen Systeme, deren Farbwert der Helligkeit L₁ nicht weniger als 50% und nicht mehr als 200% des Helligkeits-Farbwertes L₂ beträgt.

## Claims

1. Medical active substance patch comprising a matrix of monolayer or multilayer configuration as well as a backing layer connected with said matrix, wherein at least one layer of the matrix contains active substance, and wherein said active substance patch is **characterized in that**
- at least one layer of the matrix contains one or more ingredient(s) which is/are coloured, or which is/are colourless in its/their initial state and which has/have a tendency to discolour or which discolour(s) during storage or during the application period,
- it is transparent or at least translucent,
- it comprises one or more substance(s) selected from the group of the dyes and pigments in at least one of the layers mentioned,
- in the state of having been applied to a first person's skin the said patch, at a place of the skin covered with the patch, has a lightness colour value L₁ which is not less than 50% and not more than 200% of a lightness colour value L₂, with L₂ being the lightness value of the region of the skin of the same person which surrounds the applied patch, and
- that the same applies in respect of the skin of a second or any other person, provided that L₂ is in the range from 5° to 100°, especially in the range from 20° to 90°.

2. Active substance patch according to claim 1, **characterized in that** the lightness colour value L₂ of the said first person is the lightness colour value of a person of light, Caucasian skin colour, and that the lightness colour value L₂ of the said second person is the lightness colour value of a person of dark, Negroid skin colour, or vice versa.

3. Active substance patch according to claim 1 or 2, **characterized in that** it contains the said substance(s) selected from the group of the dyes and pigments in the matrix layer or in at least one of the matrix layers.

4. Active substance patch according to any one of the preceding claims, **characterized in that** on the side averted from the skin the backing layer of the patch is covered with a coating, in particular a lacquer, containing a dye or dyes or/and a pigment or pigments.

5. Active substance patch according to any one of the preceding claims, **characterized in that** at least that surface of the backing layer which is averted from the skin has reduced reflection properties.

6. Active substance patch according to claim 5, **characterized in that** the reduction in reflection properties is accomplished by means of physical methods.

7. Active substance patch according to any one of the preceding claims, **characterized in that** on the side of the backing layer which is averted from the skin there is applied an antireflection layer which preferably contains an optical dulling agent or a combination of at least two optical dulling agents.

8. Active substance patch according to claim 7, **characterized in that** said antireflection layer additionally contains at least one substance selected from the group of the dyes and pigments.

9. Active substance patch according to any one of the preceding claims, **characterized in that** the said ingredient which is colourless in its initial state and which has a tendency to discolour or which discolour(s) during storage or during the application period is a pharmaceutical active substance, particularly nicotine.

10. Active substance patch according to any one of the preceding claims, **characterized in that** it is a transdermal therapeutic system.

11. Process for the production of an active substance patch according to any one of the preceding claims, **characterized in that** said process comprises the following steps:
a) producing a system comprising a mono- or multilayer active substance-containing matrix and a backing layer connected therewith, wherein the matrix is produced using a matrix polymer or matrix polymers, an active substance or active substances and auxiliary agents, and wherein one or more substance(s) selected from the group of the dyes and pigments is/are incorporated into the matrix or/and the backing layer;
b) producing at least one further system according to step (a), this system being different in terms of the concentration of the dyes or/and pigments, and/or in terms of the type of the dyes or/and pigments used;
c) producing surface sections or punched pieces from the systems obtained in steps (a) and (b);
d) producing or providing colour charts having lightness colour values L₂ in the range from 5° to 100°, particularly in the range from 20° to 90°,
e) applying or affixing the sections or systems obtained in step (c) to the colour charts mentioned in (d);
f) measuring the colour values of the lightness L₁ of the systems located on the colour charts and determining the difference between L₂ and L₁ in each particular case;
g) selecting those systems with a colour value of the lightness L₁ which is not less than 50% and not more than 200% of the lightness colour value L₂.

## Revendications

1. Pansement médical à substance active, qui présente une matrice à une ou plusieurs couches ainsi qu'une couche arrière assemblée avec cette matrice, au moins une couche de la matrice contenant une substance active et le pansement à substance active étant **caractérisé**
- **en ce qu**'au moins une couche de la matrice contient un ou plusieurs constituants, qui présente(nt) une couleur ou qui est/sont incolore(s) dans l'état de départ et qui tend(ent) à se colorer ou qui se colore(nt) pendant l'entreposage ou pendant la durée d'application,
- en ce qu'il est transparent ou du moins translucide,
- en ce qu'il contient dans au moins une des couches mentionnées une ou plusieurs substances choisies dans le groupe des colorants et des pigments,
- en ce que le pansement, dans l'état de l'application sur la peau d'une première personne présente, sur un endroit de la peau recouvert par le pansement, une valeur colorimétrique du degré de luminosité L₁ qui n'est pas inférieure à 50% et pas supérieure à 200% d'une valeur colorimétrique du degré de luminosité L₂, L₂ étant le degré de luminosité au niveau de la région de la peau entourant le pansement appliqué de la même personne, et
- en ce que la même chose vaut en ce qui concerne la peau d'une deuxième personne ou de toute autre personne, à condition que L₂, pour toutes les personnes mentionnées, se situe dans la plage de 5° et 100°, en particulier dans la plage de 20° et 90°.

2. Pansement à substance active selon la revendication 1, **caractérisé en ce que** la valeur colorimétrique du degré de luminosité L₂ de la première personne mentionnée est la valeur colorimétrique du degré de luminosité d'une personne présentant une couleur de peau claire, caucasienne et **en ce que** la valeur colorimétrique du degré de luminosité L₂ de la deuxième personne mentionnée est la valeur colorimétrique du degré de luminosité d'une personne présentant une couleur de peau foncée, négroïde, ou inversement.

3. Pansement à substance active selon la revendication 1 ou 2, **caractérisé en ce qu'**il contient la/les substance(s) choisie(s) dans le groupe des colorants et des pigments dans la couche de la matrice ou dans au moins une des couches de la matrice.

4. Pansement à substance active selon l'une quelconque des revendications précédentes, **caractérisé en ce que** la couche arrière du pansement est revêtue sur la face dos à la peau par un revêtement contenant un/des colorant(s) et/ou pigment(s) coloré(s), en particulier une laque.

5. Pansement à substance active selon l'une quelconque des revendications précédentes, **caractérisé en ce qu'**au moins la surface dos à la peau de la couche arrière présente des propriétés de réflexion diminuées.

6. Pansement à substance active selon la revendication 5, **caractérisé en ce que** la diminution des propriétés de réflexion est réalisée par des procédés physiques.

7. Pansement à substance active selon l'une quelconque des revendications précédentes, **caractérisé en ce qu'**une couche antireflet est appliquée sur la face dos à la peau de la couche arrière, qui contient de préférence un agent de matage optique ou une combinaison d'au moins deux agents de matage optiques.

8. Pansement à substance active selon la revendication 7, **caractérisé en ce que** la couche antireflet contient en outre au moins une substance qui est choisie dans le groupe des colorants et des pigments.

9. Pansement à substance active selon l'une quelconque des revendications précédentes, **caractérisé en ce que** le constituant mentionné, qui est incolore dans l'état de départ et qui tend à se colorer ou qui se colore pendant l'entreposage ou pendant la durée d'application est une substance active pharmaceutique, en particulier la nicotine.

10. Pansement à substance active selon l'une quelconque des revendications précédentes, **caractérisé en ce qu'**il s'agit d'un système thérapeutique transdermique.

11. Procédé pour la réalisation d'un pansement à substance active selon l'une quelconque des revendications précédentes, le procédé présentant les étapes suivantes :
a) préparation d'un système, comprenant une matrice à une ou plusieurs couches, contenant une substance active et une couche arrière qui y est reliée, la matrice étant réalisée en utilisant un/des polymère(s) de matrice, une/des substance(s) active(s) et des adjuvants, et une ou plusieurs substance(s) choisie(s) dans le groupe des colorants et des pigments étant incorporée(s) dans la matrice et/ou la couche arrière ;
b) préparation d'au moins un autre système selon l'étape (a), ce système étant différent en ce qui concerne la concentration en colorants et/ou en pigments et/ou en ce qui concerne le type de colorants et/ou de pigments utilisés ;
c) préparation de sections planes ou des pièces découpées à partir des systèmes obtenus dans les étapes (a) et (b) ;
d) préparation ou mise à disposition de planches de couleur, qui présentent des valeurs colorimétriques du degré de luminosité L₂ dans la plage de 5° et 100°, en particulier dans la plage de 20° et 90° ;
e) application ou collage des sections ou systèmes obtenus dans l'étape (b) sur les planches de couleur mentionnées au point (c) ;
f) mesure de la valeur colorimétrique du degré de luminosité L₁ des systèmes se trouvant sur les planches de couleur et détermination de la différence entre L₂ et L₁, dans chaque cas particulier,
g) choix des systèmes dont la valeur colorimétrique du degré de luminosité L₁ n'est pas inférieure à 50% et pas supérieure à 200% de la valeur colorimétrique du degré de luminosité L₂.
